# EUROPEAN PATENT APPLICATION

(11) **EP 2 140 899 A1**
(43) Date of publication of application: **06.01.2010**
(21) Application number: 08752064.9
(22) Date of filing: 24.04.2008
(51) Int. Cl.: A61M 5/32, A61G 12/00, A61J 3/00, A61M 5/28

(54) **INJECTOR DOUBLING AS CONTAINER**

(30) Priority: 24.04.2007 JP 2007114051
(71) Applicant: ARTE CORPORATION, Shiyoda-ku Tokyo 101-0032 (JP)
(72) Inventor: KAKIUCHI, Makoto, Takahagi-shi Ibaraki 318-0004 (JP)
(74) Representative: Zonneveld, Hendrik Jan
(86) International application number: PCT/JP2008/057975
(87) International publication number: WO 2008/133305

(57) **Abstract**

The combined container-syringe 1 has a cylindrical cylinder 7 that is filled with a drug solution and an injection needle is mounted on the front end thereof; an engagement portion 13 that is provided at the front end side of the cylinder; a finger grip 9 that is provided on the rear end side of the cylinder 7; and a cylindrical-shaped safety device 3 that is mounted in a slidable manner on the outer periphery of the cylinder7 , and covers all the sphere of the injection needle 10 when it has moved in the front end direction. Furthermore, a return prevention stopper 27 which flexes inward in the radial direction by a pressing ring 5 and prevents the movement of the safety device 3 in the rear end direction by engaging with the engagement portion 13, and a slip-out prevention stopper 25 which prevents the movement of the safety device 3 in the front end direction, by engaging with the engagement portion 13.

## Description

### TECHNICAL FIELD

The present invention relates to a combined container-syringe that is provided with a safety device that is used for covering the injection needle after the completion of injection and the like and ensuring safety.

Priority is claimed on Japanese Patent Application No. 2007-114051, filed April 24, 2007, the content of which is incorporated herein by reference.

### BACKGROUND ART

Since a combined container-syringe can be used immediately after being taken out from the packaging without performing troublesome procedures at medical institutions due to the fact that the drug solution has been prefilled, it is very convenient and very useful for lightening the workload of people involved in medical service such as physicians and nurses. For this reason, it is being adopted by many medical facilities.

In the past, when people involved in medical service used syringes, they sometimes accidentally stuck themselves with the injection needle after being used for injection, and as a result of doing so faced the risk of a viral infection. For that reason, with the aim of safety treatment of injection needles after injection into patients, a syringe has been proposed that is equipped with a safety device as a cylindrical cover that prevents careless contact with the injection needle by covering the injection needle therewith after injection. For example, according to the syringe that is disclosed in a patent document 1, an inner circumferential wall surface of a rear end of the safety device and an outer peripheral portion of an engagement portion which is provided at a front end side of the syringe are engaged and frictionally fixed each other each other at a position that covers the injection needle. [Patent Document 1]: Japanese Unexamined Patent Application, Publication No.

### DISCLOSURE OF INVENTION

### [PROBLEMS THAT THE INVENTION IS TO SOLVE]

However, in the syringe that is equipped with the conventional safety device as described above, since the safety device is merely engaged with the engagement portion by the friction therebetween, the safety device cannot be fixed securely to the engagement portion and stability of the safety device becomes low. Moreover, according to circumstances, there is the problem that the safety device may slip out from the syringe.

The present invention has been achieved in view of the above circumstances, and has as its object to provide a combined container-syringe that is equipped with a safety device which is reliably fixed to the syringe and reliably covers an injection needle after injection into a patient so as to prevent careless removal of the safety device.

### [MEANS FOR SOLVING THE PROBLEMS]

In order to solve the abovementioned problem, this invention proposes the following means.

The combined container-syringe according to the present invention has a cylindrical cylinder that is filled with a drug solution and an injection needle is mounted on the front end thereof; an engagement portion that is provided at the front end side of the cylinder; a finger grip that is provided on the rear end side of the cylinder; and a cylindrical-shaped safety device that is mounted in a slidable manner on the outer periphery of the cylinder, and covers all the sphere of the injection needle when it has moved in the front end direction. Furthermore, a return prevention stopper that engages with the engagement portion at the position where the safety device covers all the sphere of the injection needle and prevents the movement of the safety device in the rear end direction, and a slip-out prevention stopper that is formed further to the rear end side than the return prevention stopper and engages with the engagement portion at the position where the safety device covers all the sphere of the injection needle and prevents the movement of the safety device in the front end direction, are provide on an inner circumferential wall of the safety device.

When the safety device that is engaged with the outer periphery of the cylinder is moved to the front end direction, the return prevention stopper abuts the engagement portion, the return prevention stopper flexes due to the engagement portion, and the return prevention stopper passes over the engagement portion while expanding the inner diameter thereof. When the safety device moves the position where the safety device covers all the sphere of the injection needle, the slip-out prevention stopper engages with the engagement portion, and the further movement of the safety device in the front end direction is prevented. In this state, even when attempting to move the safety device in the rear end direction, since the return prevention stopper and the engagement portion engage, such movement is prevented. That is, once the safety device covers the injection needle, the safety device is completely fixed to the combined container-syringe because the engagement portion is sandwiched between the slip-out prevention stopper and the return prevention stopper from the front end side and the rear end side. As a result, the safety device does not separate from the combined container-syringe, and neither does it move in the rear end direction and cause the injection needle to be exposed.

Furthermore, in the combined container-syringe according to the present invention, it is preferable that the return prevention stopper or the slip-out prevention stopper can deform elastically, a pressing ring is mounted in a slidable manner on the outer periphery of the safety device, and the return prevention stopper or the slip-out prevention stopper which is pressed by the pressing ring projects to the inner side in the radial direction.

The return prevention stopper or the slip-out prevention stopper does not project to the inner side in the radial direction under normal conditions, and it projects to the inner side in the radial direction only when it is pressed by the pressing ring from the outer side in the radial direction. Therefore, when mounting the safety device onto a combined container-syringe body from the front side thereof, the return prevention stopper or the slip-out prevention stopper does not abut the engagement portion by providing the stopper in the state that the stopper does not project to the inner side. Accordingly, the safety device can be mounted on the combined container-syringe body easily without preventing its movement.

Furthermore, it is preferable that the return prevention stopper has a sloping surface which is formed so as to project from the inner surface of the safety device toward the rear end direction and the slip-out prevention stopper has a sloping surface which is formed so as to project from the inner surface of the safety device toward the front end direction.

According to this, when the safety device is moved to the front end direction of the combined container-syringe body, the sloping surface of the return prevention stopper abuts the engagement portion and easily flexes the inner circumference of the safety device. Thereby, the return prevention stopper can readily pass over the engagement portion.

On the other hand, when the safety device is mounted onto the combined container-syringe body from the front end direction, the sloping surface of the slip-out prevention stopper abuts the engagement portion and easily flexes the inner circumference of the safety device. Thereby, the slip-out prevention stopper can readily pass over the engagement portion.

### [EFFECTS OF THE INVENTION]

According to the combined container-syringe of to the present invention, since the return prevention stopper and the slip-out prevention stopper of the safety device engage with the engagement portion of the combined container-syringe body, and the safety device is reliably fixed to the combined container-syringe, it is possible to reliably cover the injection needle after injection into a patient with the safety device, and so possible to ensure safety.

### BRIEF DESCRIPTION OF THE DRAWINGS

FIG. 1 is a partial sectional view of the combined container-syringe according to one embodiment of the present invention.
FIG. 2 is an enlarged view of the vicinity of the engagement portion, the return prevention stopper, and the slip-out prevention stopper in FIG. 1.
FIG. 3 is a vertical sectional view of the combined container-syringe body.
FIG. 4 is a vertical sectional view of the safety device body.
FIG. 5 is a sectional view along a line A-A in FIG. 4.
FIG. 6 is a sectional view along a line B-B in FIG. 4.
FIG. 7 is a sectional view along a line C-C in FIG. 4.
FIG. 8 is a view along arrow D in FIG. 4 in the vicinity of the return prevention stoppers.
FIG. 9A is a side view of the pressing ring.
FIG. 9B is a plan view of the pressing ring.
FIG. 10 is a vertical sectional view of the safety device.
FIG. 11A is a drawing that explains a procedure for mounting the safety device on the combined container-syringe body.
FIG 11B is a drawing that explains a procedure for mounting the safety device on the combined container-syringe body.
FIG. 11C is a drawing that explains a procedure for mounting the safety device on the combined container-syringe body.
FIG. 12 is a drawing that explains a mechanism for fixing the safety device on the combined container-syringe body according to the fourth modification embodiment of the present invention.
FIG. 13 is a sectional view in the vicinity of the return prevention stoppers according to the fifth modification embodiment of the present invention.

### [BRIEF DESCRIPTION OF THE REFERENCE SYMBOLS]

1...combined container-syringe, 2...combined container-syringe body, 3... safety device, 4... safety device body, 5...pressing ring, 7...cylinder, 8...cylindrical tip, 9...finger grip, 10...injection needle, 13...engagement portion, 25...slip-out prevention stopper, 27...return prevention stopper

### BEST MODE FOR CARRYING OUT THE INVENTION

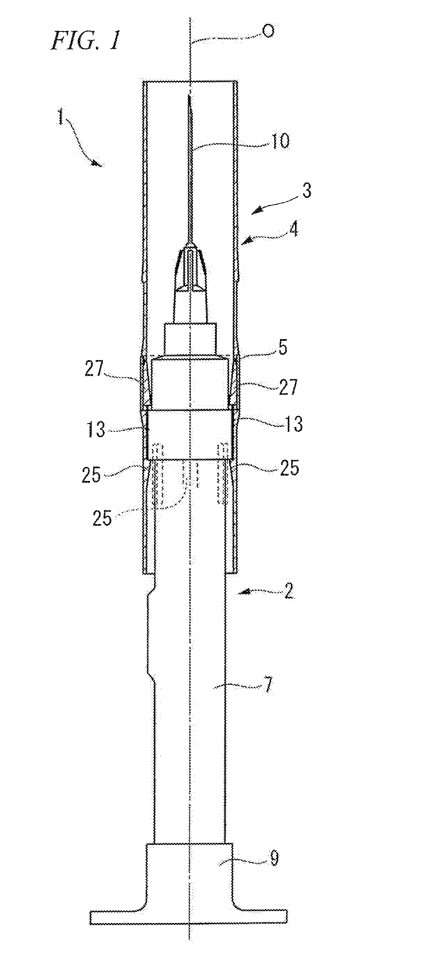

An embodiment of the combined container-syringe according to the invention shall be described hereinbelow with reference to FIG. 1 to FIG. 13.

As shown in FIG. 1, a combined container-syringe 1 in the present embodiment is constituted by a combined container-syringe body 2; a safety device 3 that consists of a safety device body 4 and a pressing ring 5. Also, hereinbelow, in FIG. 1 the upper side along an axis O of the combined container-syringe body 2 at which an injection needle 10 is provided shall be called the front end, and the lower side at which a finger grip 9 is provided shall be called the rear end.

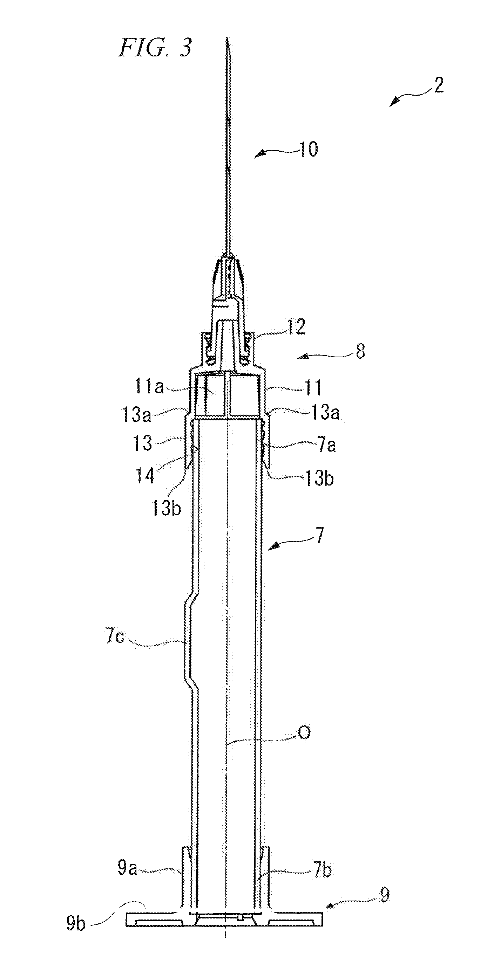

As shown in FIG. 3, the combined container-syringe body 2 is constituted by a cylinder 7, a cylindrical tip 8 that is fitted on the outer periphery of the front end side of the cylinder 7, the finger grip 9 made of a synthetic resin that is fitted on the outer periphery of the rear end side of the cylinder 7, and the injection needle 10 that is mounted on a luer-lock portion 12 that is provided at the front end of the cylindrical tip 8.

The cylinder 7 is made for example of transparent glass, and has a cylindrical shape that extends along the axis O. In the cylinder 7, a bypass path 7c is formed that bulges outward along a predetermined length in the axis direction, and a stopper (not illustrated) is provided at a total of three locations, namely, the front end side and rear end side of the cylinder 7 and directly on the rear end side of the bypass path 7c. A drug solution is sealed inside of the cylinder 7. Also, the front end portion of a plunger rod (not illustrated) that is inserted from the rear end side of the cylinder 7 is connected by being screwed into the stopper on the rear end side.

The cylindrical tip 8 is for example made with a transparent synthetic resin. As shown in FIG. 3, the cylindrical tip 8 is provided with a cylinder portion 11 in which a bypass chamber 11a having an inner diameter that is equal to or slightly larger than the inner diameter of the cylinder 7 and a stopper (not illustrated) is inserted therein, a cylindrical luer-lock portion 12 that is integrally formed at the front end of the cylinder portion 11 and has an inner hole which acts as an attachment portion of the injection needle 10 which communicates with the bypass chamber 11a, and a cylindrical engagement portion 13 that is integrally formed at the rear end of the cylinder portion 11 and has a larger diameter than it. By fitting an inner hole 14 of the engagement portion 13 on the outer periphery of the front end portion 7a of the cylinder 7, the cylindrical tip 8 is fitted on the cylinder 7. Also, the front end side of the engagement portion 13 at the cylindrical tip 8 is fashioned into an engagement step portion 13a at which a step portion is formed, and the rear end side is made into an engagement end portion 13b.

The finger grip 9 has a fitting portion 9a having a cylindrical shape and a flange 9b that is integrally formed on the rear end portion of the fitting portion 9a and extends to the outer side in the radial direction. The fitting portion 9a is fitted on the outer periphery of the rear end 7b of the cylinder 7.

Since the drug solution is filled in advance in the combined container-syringe body 2 having the above constitution, at the time of use it can be used immediately after being taken out from the packaging. Also, the safety device 3 that consists of the safety device body 4 and the pressing ring 5 is mounted on the outer periphery of the combined container-syringe body 2.

The safety device body 4 is made with a material that is transparent and has moderate flexibility such as polypropylene and thinly fabricated in a range that can maintain strength. As shown in FIG. 1, the safety device body 4 has an approximately cylindrical shape with the same axis O as the combined container-syringe body 2 serving as the longitudinal direction.

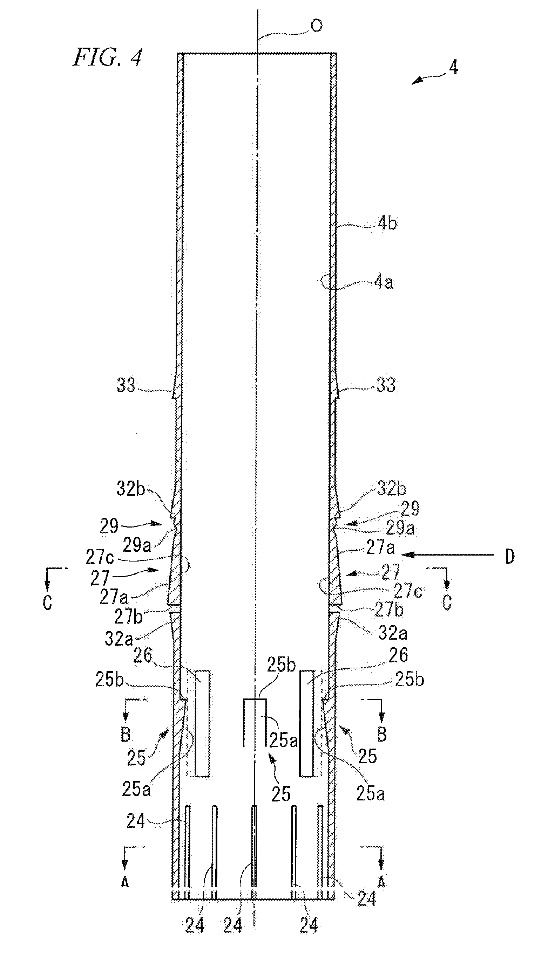

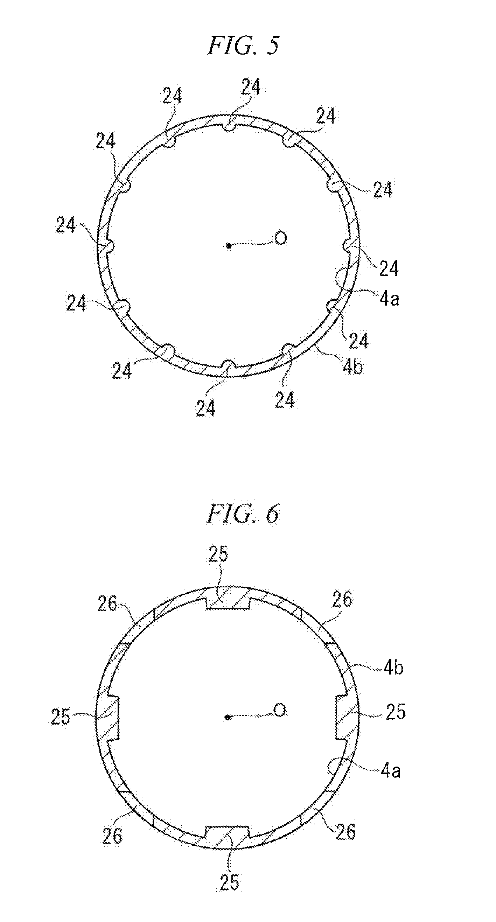

As shown in FIGS. 4 and 5, on an inner circumferential wall 4a, ribs 24 which extending along the axis O and having convex cross-section projecting to the inner side of the radial direction are provided so as to extend predetermined length from its rear end toward the front end side. These ribs 24 are uniformly spaced in the circumferential direction, and the total number of the ribs is twelve, for example.

Also, as shown in FIG. 6, slip-out prevention stoppers 25 are provided at a total of four locations on the inner circumferential wall 4a uniformly spaced in the circumferential direction, and further to the front end side than the ribs 24. Each slip-out prevention stopper 25 has a sloping surface 25a that is formed so as to gradually reduce the diameter of an inner circumferential wall 4a toward the front end and an engagement surface 25b that is perpendicular to the inner circumferential wall 4a, extending from the apex of the sloping surface 25a to the inner circumferential wall 4a. Furthermore, a total of four slits 26 are provided at intermediate positions between every two adjacent slip-out prevention stoppers 25 along the circumferential direction of the inner circumferential wall 4a, with each slit 26 extending along the axis O and opening so as to make the inner circumferential wall 4a communicate with the outer circumferential wall 4b of the safety device body 4.

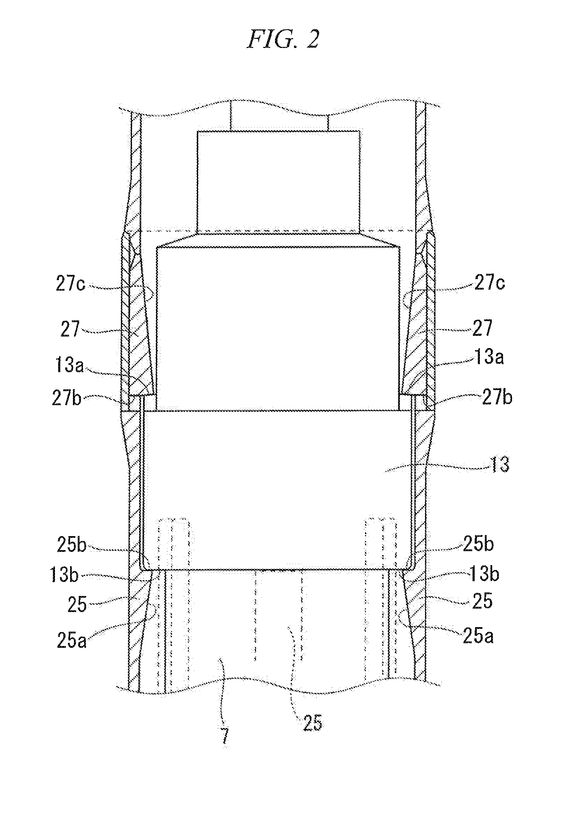

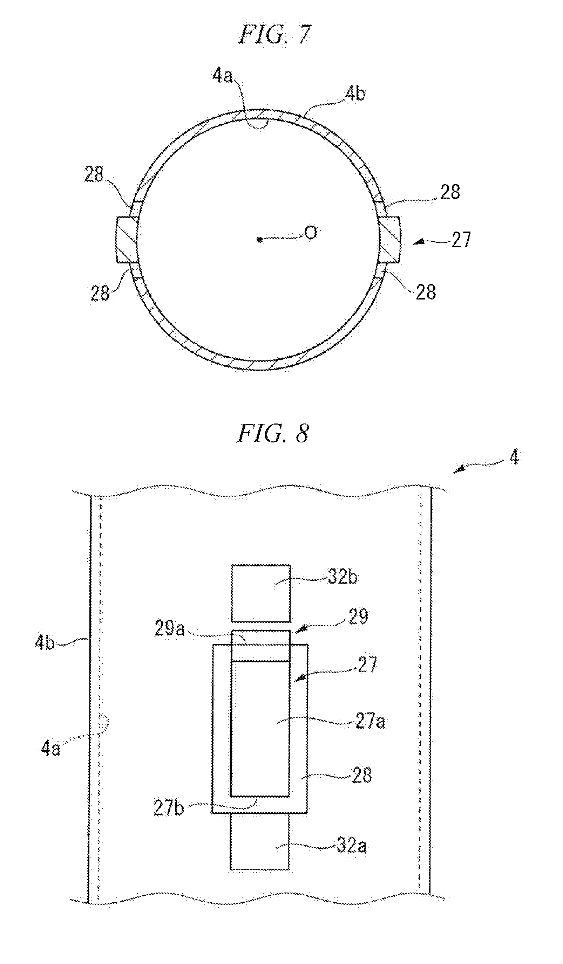

Moreover, as shown in FIG. 2, return prevention stoppers 27 are provided on the outer circumferential wall 4b of the safety device body 4 further to the front end side than the slip-out prevention stopper 25 and the slits 26. These return prevention stoppers 27 are provided at two locations so as to be provided at opposing positions as shown in FIG. 7. Each return prevention stopper 27 has an outer circumferential surface 27a that slopes so as to expand the diameter of the outer circumferential wall 4b toward the rear end direction, an engagement surface 27b that extends from the outer circumferential surface 27a to the inner side in the radial direction, and an inner circumferential surface 27c that is positioned on the reverse side of the outer circumferential surface 27a so as to be flush with the inner circumferential wall 4a of the safety device body 4.

As shown in FIG. 8 which is a view along arrow D in FIG. 4 in the vicinity of the return prevention stoppers, each return prevention stopper 27 is surrounded on the rear end side and both sides of the outer circumferential surface 27a in the circumferential direction by a U-shaped slit 28 that is cut out so as to make the inner circumferential wall 4a communicate with the outer circumferential wall 4b. By this U-shaped slit 28, the return prevention stopper 27 has a structure which is connected with the outer circumferential wall 4b of the safety device body 4 only at the front end side of the outer circumferential surface 27a, and a recessed groove 29a is formed at this connection position 29. As a result, when a force is applied to the return prevention stopper 27 from the outer side in the radial direction, the return prevention stopper 27 is flexed in the manner of a flat spring toward the inner side of the safety device body 4, with the recessed groove 29a of the connection position 29 serving as a fulcrum point.

Ring secondary stop projections 32a, 32b are provided at the rear end side of the U-shaped slit 28 and the front end side of the connection position 29, respectively, at each return prevention stopper 27 so as to sandwich the return prevention stopper 27 from the front end side and the rear end side. The ring secondary stop projection 32b that is positioned at the front end side has a slope that is formed so as to increase the diameter of the outer circumferential wall 4b toward the rear end direction. Also, two ring primary stop projections 33 that project to the outer side in the radial direction are provided at opposing positions in the circumferential direction on the outer circumferential wall 4b of the safety device body 4, being positioned further to the front end side than the front end side ring secondary stop projection 32b.

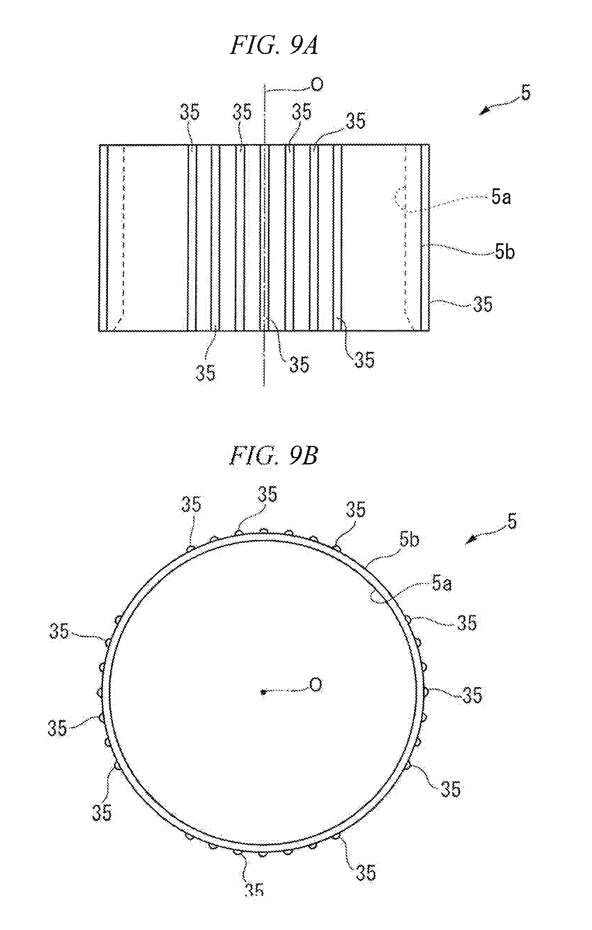

The pressing ring 5, as shown in FIGS. 9A and 9B, has a cylindrical shape in which the length in the axis O direction is sufficiently shorter than the safety device body 4, and similarly to the safety device body 4, the pressing ring 5 is made with a material that is transparent and has moderate flexibility such as polypropylene and thinly fabricated in a range that can maintain strength. A rear end side of an inner circumferential wall 5a of the pressing ring 5 has a sloped shape so as to increase the diameter of its inner circumferential wall. Furthermore, on the inner circumferential wall 5a, a plurality of vertical ribs 35 for preventing slippage are formed so as to extend along the axis O and to project toward the outer side in the radial direction. Note that the internal diameter of the pressing ring 5 is approximately the same as or slightly larger than the outer diameter of the safety device body 4. Also, in order to increase flexibility in the pressing ring 5, slits may be provided along the axis O.

Next, the method of assembling and method of using the combined container-syringe 1 shall be described.

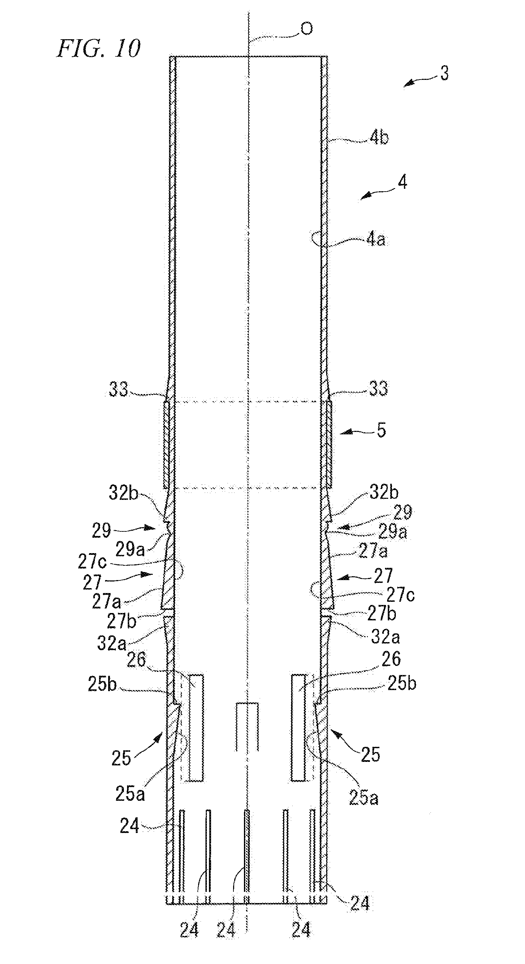

First, the inner circumferential wall 5a of the pressing ring 5 is fitted and slid on the outer circumferential wall 4b of the safety device body 4 from the front end side toward the rear end. The pressing ring 5 passes over the ring primary stop projections 33 due to its flexibility and the rear end of the pressing ring 5 makes contact and stops on the sloped surface of the ring secondary stop projection 32b. In this state, as shown in FIG. 10, further movement of the rear end side of the pressing ring 5 is prevented by the sloping surface of the ring secondary stop projection 32b, and further movement of the front end side is prevented by the ring primary stop projections 33. Thereby, the pressing ring 5 is supported by the safety device body 4 in a manner of not being moved with a slight force, and thus the safety device 3 is constituted.

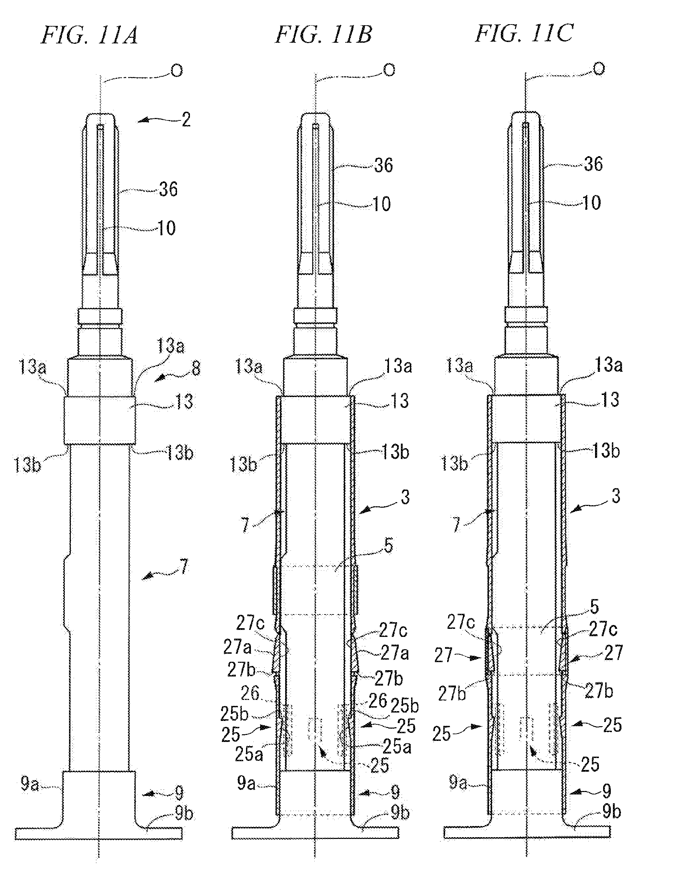

The safety device 3 is, as shown in FIG. 11A, mounted on the combined container-syringe body 2 in the state of a protector 36 being mounted on the injection needle 10 with the drug solution having been filled. When the rear end of the safety device 3 is slid from the front end side of the protector 36 of the combined container-syringe body 2 toward the rear end of the combined container-syringe body 2, the sloping surface 25a of the slip-out prevention stopper 25 abuts the engagement step portion 13a of the engagement portion 13. At this time, the sloping surface 25a flexes due to the engagement portion 13, and passes over the engagement portion 13 while expanding the inner diameter of the safety device 3. Note that since the flexibility is increased by the slits 26 being provided in the periphery of the slip-out prevention stopper 25, the slip-out prevention stopper 25 can readily pass over the engagement portion 13.

In this state, since there are no other projections on the inner circumferential wall 4a of the safety device body 4, the safety device 3 is readily slid until the rear end of the safety device body 4 abuts the flange 9b of the finger grip 9. As a result, the engagement of the safety device 3 to the combined container-syringe body 2 is completed. FIG. 11B shows this state. Furthermore, in this state, the ribs 24 provided on the rear side of the inner circumferential wall 4a of the safety device body 4 bite into the outer periphery of the finger grip 9 with appropriate force, and then, the safety device body 4 is fixed to the finger grip 9.

Next, when a force is applied so as to push the pressing ring 5 in the direction of the rear end, the pressing ring 5 passes over the sloping surface of the ring stop projection 32b to move in the rear end direction on the outer circumference of the safety device body 4, and moves further in the rear end direction while abutting the outer circumferential surface 27a of the return prevention stopper 27. The movement of the pressing ring 5 is stopped by contact with the ring secondary stop projection 32a from the front side. At this time, the outer circumferential surface 27a of the return prevention stopper 27 is pressed inward in the radial direction by the inner circumferential wall 5a of the pressing ring 5, and the return prevention stopper 27 flexes in the manner of a flat spring toward the inner side of the safety device body 4, with the recessed groove 29a of the connection position 29 serving as a fulcrum point. Thereby, as shown in FIG. 11C, the inner circumferential surface 27c of the return prevention stopper 27 slopes so as to cause the inner circumferential wall 4a to decrease in diameter toward the rear end direction, and projects from the inner circumferential wall 4a of the safety device body 4.

The drug solution is administered to the patient by the combined container-syringe 1 constituted as above. In the state of not mounting the protector 36 on the combined container-syringe 1 after use, when the force along the front direction of the combined container-syringe body 2 to the safety device 3, the safety device 3 is released from the finger grip 9 and moves toward the front end of the combined container-syringe body 2. At this time, the inner circumferential surface 27c of the return prevention stopper 27 abuts the engagement end portion 13b of the engagement portion 13, but since the safety device 3 itself is constituted with a flexible material, the inner circumferential surface 27c of the return prevention stopper 27 passes over the engagement portion 13 by flexing the inner circumferential surface 27c by the engagement portion 13 to expand the inner circumference of the safety device 3.

Afterward, as shown in FIG. 1, when the safety device 3 moves to a position at which the periphery of the injection needle 10 is completely covered with the safety device 3, the engagement surface 25b of the slip-out prevention stopper 25 and the engagement end portion 13b of the engagement portion 13 engage, and further movement of the safety device 3 toward the front end is prevented, as shown in detail in FIG. 2. Moreover, in this state, even when attempting to move the safety device 3 in the rear end direction, since the engagement surface 27b of the return prevention stopper 27 and the engagement step portion 13a of the engagement portion 13 engage, such movement is prevented. That is, once the injection needle 10 is covered with the safety device 3, since the engagement portion 13 is sandwiched between the slip-out prevention stopper 25 and the return prevention stopper 27 from the front end side and rear end side, the safety device 3 is completely fixed to the combined container-syringe body 2. As a result, the safety device 3 does not separate from the combined container-syringe body 2 as a result of proceeding too far to the front end side, and neither does it move in the direction of the rear end and cause the injection needle 10 to be exposed.

As described above, according to the combined container-syringe 1 of the embodiment, since the safety device 3 is strongly fixed to the combined container-syringe body 2 by engaging the engagement portion is of the container-syringe body 2 with the return prevention stopper 27 and the slip-out prevention stopper 25 of the safety device 3, the safety device 3 does not release from the combined container-syringe body 2. Therefore, it is possible to reliably cover the injection needle 10 with the safety device 3, and so possible to treat it safely after injection.

Furthermore, since the return prevention stopper does not project to the inner side in the radial direction while the safety device 3 is mounted on the combined container-syringe body 2, it does not prevent the movement of the safety device 3. As a result, the safety device 3 can be mounted on the combined container-syringe body 2 easily.

In addition, since the safety device 3 is fixed to the combined container-syringe body 2 by the engagement of the ribs 24 and the finger grip 9 while the safety device 3 is mounted on the combined container-syringe body 2, it does not interfere the treatment of injection to patients using the combined container-syringe 1.

The combined container-syringe 1 of the embodiment of the present invention was described above in detail, but some design modifications are possible without being limited to them as long as not departing from the technical ideas of the present invention.

For example, the structure that the cylinder 7 and the cylindrical tip 8 are integrally formed can be employed as the first modification embodiment of the present invention.

Furthermore, as the second modification embodiment of the present invention, the safety device 3 may be mounted on the combined container-syringe body 2 from the rear end side of the cylinder 7 together with the finger grip 9. Alternatively, the safety device 3 may be mounted on the combined container-syringe body 2 from the rear end side of the cylinder 7 in the state that the finger grip 9 is removed therefrom. According to this, even when the safety device 3 having the structure that the return prevention stopper projects to the inner side from the first, the safety device 3 may be mounted on the combined container-syringe body 2 easily without preventing its movement

Furthermore, as the third modification embodiment of the present invention, the safety device 3 having the structure that the slip-out prevention stopper 25 can deform elastically and the return prevention stopper 27 projects to the inner side, may be prepared. In this case, after the usage of the syringe, the safety device 3 may cover the injection needle 10 together with the engagement between the engagement potion 13 and the return prevention stopper 27 and the slip-out prevention stopper 25 by engaging the safety device 3 with the combined container-syringe body 2 from the front end side and by passing over the engagement portion 13 according to its elastic deformation.

In addition, in the cases of the second and third modification embodiments, the pressing ring 5 may be provided and the pressing ring 5 may push the return prevention stopper 27 and the slip-out prevention stopper 25. In the second and third modification embodiments, the moving direction of the safety device 3 is unidirectional, and therefore, the safety device 3 can be mounted more easily.

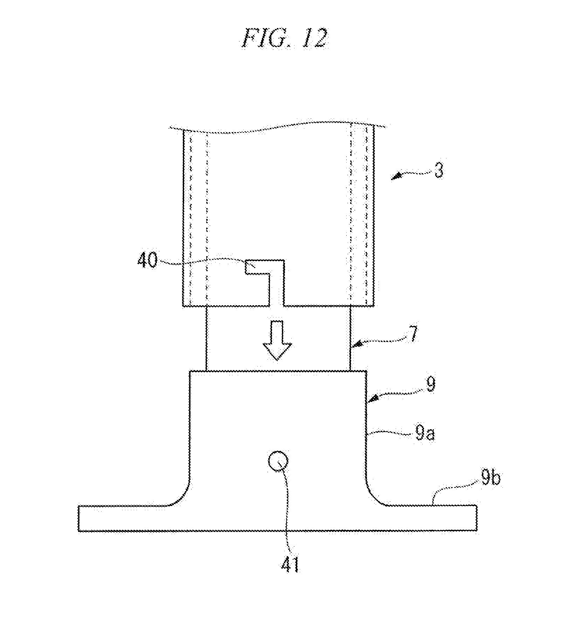

Moreover, as the fourth modification embodiment of the present invention, two L-shaped slits 40 may be formed on the rear end of the safety device 3 at opposing positions in the circumferential direction and two projections 41may be formed on the outer periphery of the fitting portion 9a of the finger grip 9 so as to project to the outer side in the radial direction at opposing positions in the circumferential direction as shown in FIG. 12. The width of the L-shaped slits 40 are the same as the width of the projections 41. In this embodiment, when the safety device 3 is mounted on the combined container-syringe body 2, the safety device 3 is fixed to the combined container-syringe body 2 by engaging two pairs of L-shaped slits 40 and projections 41 each other. Furthermore, one L-shaped slit 40 and projection 41 may be formed on the safety device 3 and the finger grip 9 respectively, and the L-shaped slit 40 and projection 41 may be engaged.

In this case, the safety device 3 is slid to the rear end side of the combined container-syringe body 2, and then the projection 41 is engaged with the portion of the L-shaped slit 40 that extends from the rear end to the front end side. When the projection 41 is abutted to the front end edge of the L-shaped slit 40, by rotating either of the safety device 3 or the combined container-syringe body 2 in the circumferential direction, engagement of the safety device 3 is performed. In this embodiment, it is needless to provide the ribs 24 to the safety device 3. According to this embodiment, it is possible to reliably fix the safety device 3 to the combined container-syringe body 2.

In addition, when the safety device is slid to the front end side of the combined container-syringe body 2, by rotating either of the safety device 3 or the combined container-syringe body 2 in the circumferential direction which is opposed to the direction for fixing them, engagement of the safety device 3 is released. According to this, the safety device 3 can be slid more easily.

Furthermore, in order to completely cover the periphery of the injection needle 10 with the safety device 3, the safety device 3 have to be slid to the front direction along the combined container-syringe body 2. In this case, for easily slid the safety device 3 toward the combined container-syringe body 2, a little clearance are provided between the inner circumferential wall 4a of the safety device 3 and the outer circumferential wall of the combined container-syringe body 2. However, due to the size of this clearance, there is the case that the safety device 3 may be shook from side to side when the periphery of the injection needle 10 is covered with the safety device 3. The fifth modification embodiment of the present invention is achieved to prevent this shake.

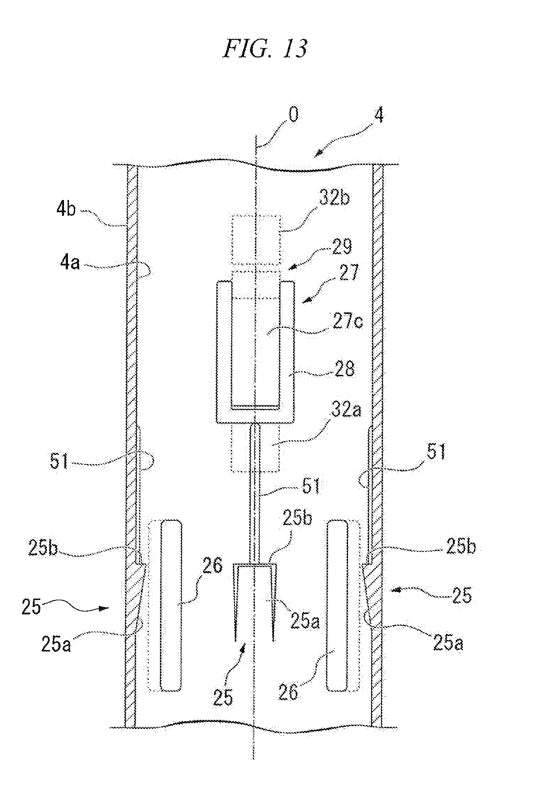

In the fifth modification embodiment, as shown in FIG. 13, a plurality of ribs 51 which extend along the axis O are formed on the inner circumferential wall 4a of the safety device body 4 so as to position between the engagement portion 25b of the slip-out prevention stopper 25 and the U-shaped slit 28 of the return prevention stopper 27 (that is the position which covers the engagement portion 13 of the combined container-syringe body 2 when the periphery of the injection needle 10 is covered with the safety device 3). These ribs 51 project to the inner side of the radial direction and are uniformly spaced in the circumferential direction. Here, the projecting amount of each rib 51 is set approximately the same as or slightly smaller than the clearance between the inner circumferential wall 4a of the safety device 3 and the outer circumferential wall of the combined container-syringe body 2. Furthermore, in FIG. 13, each rib 51 is positioned at the front end side of the engagement surface 25b, however, it is unnecessary to position the rib 51 at the front end side of the engagement surface 25b. That is, the position of the rib 51 may be deviated from the engagement surface 25b as far as the rib 51 is provided between the engagement surface 25b and the return prevention stopper 27. In addition a number of the ribs 51 are not limited as far as a plurality of ribs 21 are positioned in the circumferential direction of the inner circumferential wall 4a in a well-balanced manner and as the shake of the safety device 3 which is supported by the engagement portion 13 via the ribs 51 is prevented or reduced. That is, the number of the ribs is not less than three. Moreover, it is not necessary to equalize the space between the ribs 5 in the circumferential direction.

According to this embodiment, the safety device 3 is supported by the engagement portion 13 via the ribs 51 when the periphery of the injection needle 10 is covered with the safety device 3, and therefore, the shake of the safety device 3 is prevented or reduced. Furthermore, since the safety device 3 and the outer periphery of the engagement portion 13 are contacted via the back of the ribs 51, contact area between them is little.

In addition, since the ribs 51 are formed so as to extend along the axis O, frictional resistance between the outer periphery of the combined container-syringe body 2 and the back of the ribs 51 is also little when the safety device 3 is slid toward the combined container-syringe body 2. Therefore, easiness of the sliding performance between the safety device 3 and the combined container-syringe body 2 does not deteriorated by the presence of the ribs 51.

In the combined container-syringe 1 according to the above embodiments, the embodiments that the present invention applies to a double chamber type combined container-syringe are explained, however the present invention is not limited to them, and the present invention can be applied to a single chamber type combined container-syringe.

### INDUSTRIAL APPLICABILITY

According to the present invention, it is possible to reliably cover the injection needle with the safety device, and so possible to treat it safely after injection.

## Claims

1. A combined container-syringe comprising:
a cylindrical cylinder that is filled with a drug solution and an injection needle is mounted on the front end thereof;
an engagement portion that is provided at the front end side of said cylinder; and
a finger grip that is provided on the rear end side of said cylinder; wherein
a cylindrical-shaped safety device that is mounted in a slidable manner on the outer periphery of said cylinder, and covers all the sphere of said injection needle when it has moved in the front end direction,
a return prevention stopper that engages with said engagement portion at the position where the safety device covers all the sphere of said injection needle and prevents the movement of said safety device in the rear end direction, and
a slip-out prevention stopper that is formed further to the rear end side than said return prevention stopper and engages with said engagement portion at the position where the safety device covers all the sphere of said injection needle and prevents the movement of said safety device in the front end direction, are provide on an inner circumferential wall of the safety device.

2. A combined container-syringe according to claim 1 wherein said return prevention stopper or said slip-out prevention stopper can deform elastically, a pressing ring is mounted in a slidable manner on the outer periphery of said safety device, and said return prevention stopper or said slip-out prevention stopper which is pressed by the pressing ring projects to the inner side in the radial direction.

3. A combined container-syringe according to claim 1 wherein said return prevention stopper has a sloping surface which is formed so as to project from the inner surface of said safety device toward the rear end direction and said slip-out prevention stopper has a sloping surface which is formed so as to project from the inner surface of said safety device toward the front end direction.
